(19) Europäisches Patentamt
European Patent Office
Office européen des brevets

(11) **EP 1 081 121 B1**

(12) # EUROPEAN PATENT SPECIFICATION

(45) Date of publication and mention of the grant of the patent:
**22.05.2013 Bulletin 2013/21**

(21) Application number: **00307360.8**

(22) Date of filing: **25.08.2000**

(51) Int Cl.:
***C07B 63/00*** *(2006.01)*
***C07C 7/13*** *(2006.01)*
***C07C 17/389*** *(2006.01)*
***C07C 15/067*** *(2006.01)*
***C07C 25/08*** *(2006.01)*
***B01D 15/00*** *(2006.01)*

(54) **Method for producing aromatic compound isomers**

Verfahren zur Herstellung von Isomeren von aromatischen Verbindungen

Procédé pour la préparation d'isomères de composés aromatiques

(84) Designated Contracting States:
**AT BE CH CY DE DK ES FI FR GB GR IE IT LI LU MC NL PT SE**

(30) Priority: **26.08.1999 JP 24039399**

(43) Date of publication of application:
**07.03.2001 Bulletin 2001/10**

(73) Proprietor: **TORAY INDUSTRIES, INC.**
**Tokyo 103-8666 (JP)**

(72) Inventors:
- **Kato, Masashi**
**Nagoya-shi,**
**Aichi 458-0044 (JP)**
- **Kato, Yasuharu**
**Nagoya-shi,**
**Aichi 257-0866 (JP)**
- **Okada, Koichi**
**Tokai-shi,**
**Aichi 476-0003 (JP)**

(74) Representative: **Webster, Jeremy Mark et al**
**Mewburn Ellis LLP**
**33 Gutter Lane**
**London**
**EC2V 8AS (GB)**

(56) References cited:
**EP-A- 0 412 215**
**EP-A- 0 425 740**
**US-A- 4 962 245**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a method for producing isomers of aromatic compounds. More precisely, the invention relates to a method of adsorptive separation for producing desired isomers of aromatic compounds by the use of a zeolite-containing adsorbent and a desorbent.

Aromatic compounds are useful for starting materials for synthetic products and for intermediates for medicines and agricultural chemicals. For obtaining a specific isomer of an aromatic compound, a known method of adsorptive separation comprises contacting an isomer mixture with a zeolite-containing adsorbent.

For example, Japanese Patent Publication No. 45457/1989 discloses a method of using an adsorbent of faujasite zeolite for adsorptive separation of 2,6-dichlorotoluene; Japanese Patent Laid-Open No. 330025/1992 discloses a method of using an adsorbent of faujasite zeolite that indispensably contains potassium and lead ions, for adsorptive separation of m-dichlorobenzene; and Japanese Patent Publication No. 46933/1992 discloses a process of isomerizing halogenobenzene derivatives, in which HCl formed in the step of isomerization is first removed through distillation or the like, and a specific isomer is thereafter separated from the isomer mixture through adsorptive separation by the use of a zeolite adsorbent.

Regarding heterocyclic compounds, Japanese Patent Laid-Open Nos. 143162/1997 and 251230/1998 and EP 173,440 disclose a method of using, as an adsorbent, faujasite zeolite for adsorptive separation of a compound having a pyridine ring structure.

In the process of adsorptive separation of isomers of aromatic compounds by the use of a zeolite-containing adsorbent, the adsorbent is degraded while used continuously. In the process, therefore, the adsorbent used must be exchanged for a fresh one in good time or must be regenerated, for example, by burning it. Accordingly, a technique for prolonging the life or the regeneration cycle of adsorbent, if possible, will produce great industrial merits.

In the process of the above-mentioned Japanese Patent Publication No. 46933/1992 for adsorptive separation of an isomer of an aromatic compound by the use of a zeolite adsorbent, the step of removing the side product, HCl, from the isomer mixture prior to the next step of separating the desired isomer by the use of the adsorbent is carried out so as to preventing the adsorbent used from being degraded. However, the process still does not satisfactorily prolong the life and the regeneration cycle of the adsorbent to the industrial level.

In ordinary adsorptive separation of aromatic compound isomers, a desorbent may be used, which is generally circulated and reused. In the line, however, the desorbent circulated for reuse is often contaminated with minor impurities which already existed in the mixture of aromatic isomers of the compound(s) used as starting material and with other impurities having formed through decomposition or reaction of the isomers of the aromatic compound and/or the desorbent, because the impurities accumulate in the desorbent being used and circulated. As a result, the adsorbent so used in the line will be gradually degraded.

EP-A-425740 describes a process in which para-xylene is separated from a feed mixture of $C_8$ isomers of xylene and $C_9$ aromatics using a crystalline aluminosilicate zeolite. A para-xylene is selectively adsorbed on to the adsorbent and the non-adsorbed feed is removed by desorption with a desorbent comprising 1,2,3,4-tetrahydronaphthalene or an alkyl or dialkyl derivative thereof. The $C_9$ aromatics and the xylene isomers are recovered in the raffinate and can be separated from the desorbent by fractionation of the raffinate and the recovered desorption recycled to the process.

EP-A-0412215 describes a similar process in which diethyl toluene is used as a desorbent and the desorbent is reused in the process after removal of the $C_9$ aromatics by simple fractionation.

The present invention addresses the problem of efficiently producing isomers of aromatic compounds by adsorptive separation using a zeolite-containing adsorbent, for which the adsorbent used is prevented from being degraded and its life is prolonged.

To solve or reduce the severity of the problem, the invention provides a method for producing an isomer of an aromatic compound substituted with alkyl group(s) and/or halogen atom(s), by adsorptive separation using a zeolite-containing adsorbent and a desorbent, wherein the desorbent is, after having been processed to remove impurities from it, said impurities having a higher boiling point than the desorbent, supplied to the adsorptive separation step.

Preferred embodiments of the method of the invention are as follows:

The aromatic compound has a benzene ring or heterocyclic ring structure;
the aromatic compound is ring-substituted with at least one halogen element;
the impurities in the desorbent are removed through any of distillation, desorption or adsorption to a solid adsorbent;
the impurities in the desorbent are removed by replacing a part of the used desorbent with an impurity-free fresh desorbent; and
all or part of the desorbent to be supplied to the adsorptive separation step is first continuously or intermittently supplied to a step of removing impurities from it, and then supplied to the adsorptive separation step.

The aromatic compound to be subjected to adsorptive separation according to the invention is an aromatic compound substituted with alkyl group(a) and/or halogen atom(s). The aromatic compound includes a plurality of isomers. In case where the boiling point difference between these isomers is small, the isomers are difficult to separate from each other by distillation. For the isomers, therefore, a method suitable for separating them is by adsorptive separation.

The aromatic compound substituted with alkyl group(s) and/or halogen atom(s) to be processed according to the invention has at least one substituent selected from alkyl groups and halogen atoms, but may optionally have, in addition, any other substituent(s), for example, oxygen-containing substituents such as hydroxyl groups, aldehyde groups and carboxyl groups, and nitrogen-containing substituents such as $-NO_2$, $-NH_2$, -NHR, $-NR_2$ and -NRR' (where R and R' are at least one of methyl, ethyl and propyl groups).

The aromatic compound referred to herein is not limited to benzene derivatives only, but includes any others, for example, polycyclic aromatic compounds such as naphthalene, anthracene and phenanthrene compounds, and heterocyclic compounds such as pyridine and triazine compounds.

Specific examples of the heterocyclic compounds are α-picoline, β-picoline, γ-picoline, 3,5-lutidine, 2,6-lutidine, 2,4-lutidine, 2,5-lutidine, 3,4-lutidine, 2,3-lutidine, 2,4,6-collidine, 2-ethylpyridine, 3-ethylpyridine, 4-ethylpyridine, 2-vinylpyridine, 3-vinylpyridine, 4-vinylpyridine, 2-pyridinemethanol, 3-pyridinemethanol, 4-pyridinemethanol, 2-aminopyridine, 3-aminopyridine, 4-aminopyridine, 2-dimethylaminopyridine, 3-dimethylaminopyridine, 4-dimethylaminopyridine, 2-aldehydopyridine, 3-aldehydopyridine, 4-aldehydopyridine, nicotinic acid, isonicotinic acid, picolinic acid, 2-chloropyridine, 3-chloropyridine, 4-chloropyridine, 3,5-dichloropyridine, 2,6-dichloropyridine, 2,4-dichloropyridine, 2,5-dichloropyridine, 3,4-dichloropyridine, 2,3-dichloropyridine, 2-cyanopyridine, 3-cyanopyridine, 4-cyanopyridine, 2-chloro-5-methylpyridine, 2-chloro-4-methylpyridine, 2-chloro-3-methylpyridine, 2-chloro-6-methylpyridine, methylpyrrole, ethylpyrrole, propylpyrrole, dimethylpyrrole, chloropyrrole, methylfuran, ethylfuran, propylfuran, dimethylfuran, chlorofuran, methylthiophene, ethylthiophene, propylthiophene, dimethylthiophene, chlorothiophene, methylquinoline, ethylquinoline, propylquinoline, dimethylquinoline and chloroquinoline.

Preferred starting materials for application thereto of a method embodying the invention, are aromatic compounds substituted with alkyl group(s) and/or halogen atom(s) of the following general formula (I):

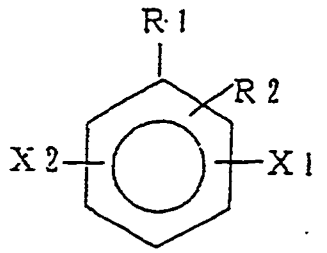

(I)

wherein R1 and R2 independently each represent any of halogen atoms and alkyl groups; and X1 and X2 independently each represent any of halogen atoms, hydrogen atoms and alkyl groups.

More preferred compounds are those that are ring-substituted with at least one halogen atom. Especially preferably, the halogen atom is chlorine or bromine. Even more preferred are aromatic compounds that are ring-substituted with chlorine atom(s).

Specific examples of the aromatic compounds (isomers) of formula (I) include xylene, trimethylbenzene, tetramethylbenzene, ethyltoluene, diethylbenzene, triethylbenzene, chlorotoluene, dichlorotoluene, trichlorotoluene, chloroethylbenzene, dichlorobenzene, trichlorobenzene, tetrachlorobenzene, bromotoluene, bromoethylbenzene, dibromobenzene, dibromotoluene, chlorobromobenzene, dibromochlorobenzene and dichlorobromobenzene. Of those, the method of the invention is especially favorable to the production of chlorotoluene, dichlorotoluene, chloroethylbenzene, dichlorobenzene and trichlorobenzene.

In a method of the invention, a zeolite-containing adsorbent is used for adsorptive separation of isomers of aromatic compounds such as those mentioned above.

The zeolite which may be employed is not limited to crystalline aluminosilicates only, and includes others, for example, crystalline aluminophosphates and silicoaluminophosphates such as ALPO and SAPO.

The adsorbent may be suitably selected, depending upon the type of the compound to be separated with it. For example, for adsorptive separation of xylene, a faujasite zeolite, for example, is preferred; but for adsorptive separation of halogenoaromatic compounds such as chloroaromatic compounds, a faujasite zeolite or pentasil zeolite, for example, is preferred. These zeolite-containing adsorbents may be ion-exchanged with, for example, alkali metal salts, alkaline earth metal salts for controlling their adsorption selectivity. For facilitating their use on an industrial scale, the adsorbents are preferably shaped along with a binder such as alumina or bentonite.

The desorbent for use in a method of the invention includes, for example, aromatic compounds such as toluene,

xylene, naphthalene, chlorobenzene and dichlorotoluene. For separating heterocyclic compounds by a method according to the invention, in addition to the above-mentioned desorbents, any of aniline, methylaniline, dimethylaniline, diphenylamine, triphenylamine, toluidine, anisidine, chloroaniline, bromoaniline, nitroaniline, dinitroaniline, trinitroaniline, phenylenediamine, methylamine, dimethylamine, ethylamine, triethylamine, n-propylamine, di-n-propylamine, tri-n-propylamine, isopropylamine, n-butylamine, isobutylamine, sec-butylamine, tert-butylamine, cyclohexylamine, benzylamine, $\alpha$-phenylethylamine, $\beta$-phenylethylamine, ethylenediamine, tetramethylenediamine, hexamethylenediamine, piperazine, piperidine and pyrrolidine may be used.

In a method of the invention, it is important to remove impurities that may be in the desorbent and will degrade the adsorbent being used, from the desorbent. Preferably, the impurity content of the desorbent to be circulated and reused in the invention is as small as possible. Specifically, it is desirable that the impurity content is at most 1000 ppm by weight, more preferably at most 100 ppm by weight. Removing impurities from the desorbent is effective for preventing the desorbent used from being degraded and for ensuring efficient adsorptive separation of aromatic compound isomers.

The method of measuring the impurities in the desorbent varies, depending upon the type of the impurities. For example, the content of impurities therein may be measured, for example, by gas chromatography, Karl Fischer's moistometry, Beckman's dissolved oxygen analysis or colorimetry under heat.

For removing the impurities from the desorbant, it may be processed, for example by distillation, desorption, or adsorption to a solid adsorbent, or a part of the used desorbent may be replaced with an impurity-free fresh desorbent.

Specifically, in embodiments, the desorbent containing impurities is distilled to remove from it impurities having a higher boiling point by evaporation. Impurities having a lower boiling point than the desorbent can also be removed by distillation. The distillation may be effected in any ordinary manner. For example, a distillation tower may be used, which may be any of a plate column tower and a packed tower, and the condition for operating it is not specifically defined.

For removing the impurities from the desorbent by desorption, for example, it is possible to employ any of a method of directly bubbling $N_2$ into the desorbent in a storage tank or a method of contacting the desorbent with $N_2$ in countercurrent streams of the two in, for example, a plate column tower or a packed tower. The condition for operation is not specifically defined.

The desorbent may be processed by adsorption to a solid adsorbent for removing impurities from it. For example, the desorbent that contains impurities is contacted with a solid adsorbent so that the impurities are selectively adsorbed by the solid adsorbent, whereby the impurities are removed from the desorbent. For contacting the desorbent with a solid adsorbent, any of, for example, a fixed bed and a fluidized bed is empoyable. The solid adsorbent includes, for example, activated charcoal, zeolite, acid clay, activated clay and alumina.

Of the impurities that may accumulate in the desorbent used in the process of adsorptive separation, highly polar substances such as water, hydrochloric acid, phenols and others strongly adsorb onto the surface of the adsorbent to lower the adsorptive separation capability of the adsorbent. Of those, dimmers of aromatic compounds will be carbonated in the adsorbent to thereby also lower the adsorptive separation capability of the adsorbent. Therefore, removing these impurities from the desorbent, if possible, will be effective for preventing the adsorbent from being degraded, whereby the regeneration cycle of the adsorbent could be prolonged. Accordingly, by a method embodying the invention, it is desirable to remove the following impurities, namely water, hydrochloric acid, phenols, dimmers of aromatic compounds, unsaturated hydrocarbon-containing compounds, and also oxygen-containing compounds such as those having aldehyde groups and carboxyl groups, from the desorbent.

In a method embodying the invention, all or part of the desorbent to be circulated and reused in the adsorptive separation step may be processed in the previous step of removing the impurities from it, and the desorbant may be supplied to the steps of removing the impurities either continuously or intermittently.

In a method embodying the invention, the adsorptive separation may be effected in any known manner. For example, it may be effected in any of a fixed bed, a fluidized bed or a moving bed, in any manner of a flow process or batch process. The isomer mixture and the desorbent to be processed for such adsorptive separation may be in a vapor phase or a liquid phase. One typical process for adsorptive separation is a simulated moving bed process.

One preferred method embodying the invention, particularly suitable for recovery of, for example, p-xylene from a mixture of ethyl benzene and o-, m- and p-xylenes, comprises the steps of introducing a starting material into an adsorption chamber through which a desorbent is flowing, withdrawing from the adsorption chamber a raffinate of unabsorbed starting material and a proportion of desorbent, withdrawing remaining desorbent and desired isomer from an end of the adsorption chamber and recirculating it to the opposite end, after passage through -a section of the adsorption chamber extracting from the adsorption chamber an extract of the desired isomer and a proportion of desorbent, subjecting each of the extract and raffinate to respective separation processes to recover the desired isomer from the extract, unabsorbed starting materials from the raffinate and respective proportions of the desorbent from each of the extract and raffinate, subjecting desorbent from the extract and raffinate to a separation process to remove impurities therefrom and recirculating the purified desorbent to the adsorption chamber.

For recovery of p-xylene from a mixture of ethylbenzene and o-, m- and p-xylenes, a preferred desorbent comprises at least 90wt.% of p-diethylbenzene.

Another preferred method embodying the invention, particularly suitable for recovery of, for example, m-dichlorobenzene from a mixture of o-, m- and p-dichlorobenzene, comprises the steps of introducing a starting material into an adsorption chamber through which a desorbent is flowing, withdrawing from the adsorption chamber a raffinate of the desired isomer and a proportion of desorbent, withdrawing remaining desorbent and starting material from an end of the adsorption chamber and recirculating it to the opposite end, after passage through a section of the adsorption chamber extracting from the adsorption chamber an extract of remaining starting material and a proportion of desorbent, subjecting each of the extract and raffinate to respective separation processes to recover the desired isomer from the raffinate, unabsorbed starting materials from the extract and respective proportions of the desorbent from each of the extracts and raffinate, subjecting desorbent from the extract and raffinate to a separation process to remove impurities therefrom and recirculating the purified desorbent to the adsorption chamber.

For recovery of m-dichlorobenzene from a mixture of o-, m- and p-dichlorobenzene, a preferred desorbent is 3,4-dichlorotoluene.

Especially preferred embodiments of the invention will now be described in more detail with reference to the following Examples and accompanying drawings in which:

Fig. 1 is a flow chart illustrating a method embodying the invention for providing an aromatic compound, including removal of impurities from the desorbent in the simulated moving bed process of Example 1.

Fig. 2 is a graph of adsorption recovery versus running time for the one third of Example I and Comparative Examples 1 and 2.

Fig. 3 is a chart of high-boiling-point impurities accumulated in desorbent in the method of Example 1.

Fig. 4 is a flow chart illustrating an alternative method embodying the invention, including removal of impurities from the desorbent in the simulated moving bed process of Example 2.

Fig. 5 is a graph of adsorption recovery versus running time in the method of Example 2 and Comparative Examples 3 and 4.

Fig. 6 is a chart of high-boiling-point impurities in the distillate from the distillation unit and the bottom liquid remained in the unit in the method of Example 2.

Fig. 7 is a graph of adsorption recovery versus running time in the methods of Example 3 and Comparative Example 5.

Example 1:

Na-Y type zeolite having a molar ratio $SiO_2/Al_2O_3$ of 4.8 (from Shokubai Kasei) is prepared according to the method described in the Examples in Japanese Patent Laid-Open No. 15833/1973, and ion-exchanged into an adsorbent of K-Y type zeolite. Using this, paraxylene (hereinafter referred to as PX) is separated from a mixture essentially comprising xylene isomers and ethylbenzene (this is a starting mixture to be subjected to adsorptive separation herein, and is hereinafter referred to as a starting mixture). For the system to be used herein, the simulated moving bed system described in Example 5 in Japanese Patent Laid-Open No. 330025/1992 is so modified that it has 24 adsorption chambers. The outline of the system used herein is shown in Fig. 1; the conditions for adsorptive separation in the system are given in Table 1; and the composition of the starting mixture is given in Table 2. In this system, the desorbant used is at least 90 wt.% paradiethylbenzene (hereinafter referred to as p-DEB) and the starting mixture is subjected to adsorptive separation. The desired product, PX, is taken out as an extract flow along with the desorbent; and the remaining PX not having been recovered in the extract flow and the other components of the starting mixture are taken out as a raffinate flow along with the desorbent. The extract and the raffinate are then processed in the distillation units 1 and 2, respectively, to recover the desorbent. The thus-recovered desorbent is circulated and reused in the system along with the desorbent also recovered from the 24th adsorption chamber. When contacted with the adsorbent at high temperatures in the system, the starting mixture and the desorbent will form high-boiling-point substances such as dimmers; or when contacted with a minor amount of $O_2$ in the $N_2$ streams in the distillation units, they will form oxygen-containing compounds having an aldehyde group or a carboxyl group. Though their amount is small, these impurities will accumulate in the desorbent to be circulated and reused in the system whereby the adsorptive separation capability of the system is lowered.

An index of the separation capability of the system is the "adsorption recovery" for 99.80 wt.% product purity to be defined by the following equation, and its time-dependent change is plotted, based on the adsorption recovery at the start of the system.

Adsorption Recovery

= [(amount of PX recovered as product, g/hr)/(amount of PX in Xylene isomer mixture processed for adsorptive separation, g/hr)] x 100

In this Example, a part of the desorbent is continuously extracted out and processed in the distillation unit 3 to remove the high-boiling-point substances from it, and then circulated and reused in the system. Accordingly, as shown in Fig. 2, the adsorption capability of the system is not lowered. On the other hand, the high-boiling-point substances having been removed from the desorbent are concentrated and analyzed by gas chromatography, for which the conditions are as shown in Table 3. From the chart of the gas chromatogram shown in Fig. 3, it is seen that many high-boiling-point substances are in the desorbent.

Comparative Example 1:

The same absorptive separation process as in Example 1 is repeated herein. In this, however, the step of continuously extracting a part of the desorbent to be circulated and reused in the system, and processing it for removing impurities therefrom through distillation is omitted. The adsorption recovery in the system is greatly lowered, as shown in Fig. 2.

Comparative Example 2:

Firstly, the adsorptive separation process of Comparative Example 1 is repeated for a while, but halfway through it, this is combined with the step of desorbent purification as in Example 1. In this, the adsorptive recovery in the system is greatly lowered before the halfway stage, but, as shown in Fig. 2 the reduction in the adsorptive recovery is stopped after the start of the step of removing impurities,

Example 2:

A zeolite adsorbent for separation of m-dichlorobenzene (hereinafter referred to as m-DCB) is prepared according to the method described in Examples 1 to 4 in Japanese Patent Laid-Open No. 330025/1992. This is a Y-type zeolite containing cations of potassium and lead ions. The adsorbent is baked at 500 C for 2 hours, and set in a simulated moving bed system for adsorptive separation, as in Example 5 in Japanese Patent Laid-Open No. 330025/1992. The outline of the system used herein is shown in Fig. 4; and the conditions for adsorptive separation in the system are given in Table 4. In this system, 3,4-dichlorotoluene (hereinafter referred to as 3,4-DCT) is used as the desorbent, and a DCB isomer mixture is subjected to adsorptive separation. The desired product, m-DCB is taken out as a raffinate flow along with the desorbent; and the remaining m-DCB not having been recovered in the raffinate flow and the other components o-DCB and p-DCB are taken out as an extract flow along with the desorbent. The extract and the raffinate are then processed in the distillation units 1 and 2, respectively, to recover the desorbent. The thus-recovered desorbent is circulated and reused in the system along with the desorbent also recovered from the adsorption chamber 12. When heated, 3,4-DCT forms a small amount of high-boiling-point substances such as biphenylmethanes. The impurities generally accumulate in the desorbent to be circulated and reused in the system whereby the adsorptive separation capability of the system is lowered.

An index of the separation capability of the system is the adsorption recovery for 99.5 % product purity to be defined by the following equation, and its time-dependent change is plotted, based on the adsorption recovery at the start of the system.

Adsorption Recovery

= [(amount of m-DCB recovered as product, g/hr)/(amount of m-DCB in DCB isomer mixture processed for adsorptive separation, g/hr)] x 100.

In this Example, a part of the desorbent is continuously extracted out and processed in the distillation units 3 and 4 to remove the impurities from it, and then circulated and reused in the system. Accordingly, as shown in Fig. 5 the adsorption capability of the system is not lowered. On the other hand, the high-boiling-point substances having been removed from the desorbent are concentrated and analyzed through gas chromatography, for which the condition is shown in Table 5. From the chart of the gas chromatogram shown in Fig. 6, it is seen that many high-boiling-point substances are in the desorbent.

Table 1

| Item | Condition |
|---|---|
| Adsorption Chambers | 16 ml x 24 chambers |
| Amount of Desorbent fed into system | 138.7 gr/hr |
| Amount of Starting Mixture fed into system | 62.7 gr/hr |
| Amount of Extract Flow | 61.6 gr/hr |
| Amount of Raffinate Flow | 139.8 gr/hr |
| Adsorption Temperature | 175°C |
| Amount of Desorbent Flow Circulated and Reused after Distillation | 1.6 gr/hr |

Table 2

| Component | Concentration |
|---|---|
| Ethylbenzene | 7.68 wt.% |
| Paraxylene | 21.23 wt. % |
| Metaxylene | 49.19 wt. % |
| Orthoxylene | 21.90 wt. % |

Table 3

| Item | Condition |
|---|---|
| Detector | FID |
| Column | Thermol-3 (5%), Celite 545 |
| Column Temperature | 100°C → 240°C programmed temperature gas chromatography) |
| Inlet Port Temperature | 250°C |
| Time at which heating started | 240 seconds after sample introduction |
| Carrier Gas | N2 30 cc/min |
| Amount of Sample applied to column | 1 μl |

Table 4

| Item | Condition |
|---|---|
| Capacity of Adsorption Chambers | 16 ml x 12 chambers |
| Amount of Desorbant fed into system | 364 ml/hr |
| Amount of DCB Isomer Mixture fed into system | 16 ml/hr |
| Amount of Extract Flow | 71 ml/hr |
| Amount of Raffinate Flow | 37 ml/hr |
| Adsorption Temperature | 130°C |
| Time for switching simulated moving beds | about 150 seconds |
| Amount of Desorbent Flow Circulated and Reused after Distillation | 12 ml/hr |

Table 5

| Item | Condition |
|---|---|
| Detector | FID |
| Column | Silicon OV-17 (5 %)/Uniport B, 60-80 mesh, 2 m x 3 mmφ |
| Column Temperature | 250°C |
| Inlet Port Temperature | 300°C |
| Carrier Gas | N2, 225 kPa |
| Air | 50 kPa |
| Hydrogen | 60 kPa |
| Amount of Sample applied to column | 1 μl |

Comparative Example 3:

The same absorptive separation process as that in Example 2 is repeated herein. In this process, however, the step of continuously extracting apart of the desorbent to be circulated and reused in the system, and processing it for removing impurities therefrom through distillation is omitted. The adsorption recovery in the system is greatly lowered, as shown in Fig. 5.

Comparative Example 4:

Firstly, the adsorptive separation process of Comparative Example 1 is repeated for a while, but halfway through it, this is combined with the step of desorbent purification as in Example 2. In this process, the adsorptive recovery in the system is greatly lowered before the halfway stage, but as shown in Fig. 5, the reduction in the adsorptive recovery is stopped after the start of the step of removing impurities.

Example 3:

To 100 parts by weight of sodium-type Y-type zeolite (hereinafter referred to as Na-Y) (powdered product of Zeolum Na-5.1Y from Toso), is added 15 parts by weight, in terms of alumina, of alumina sol (Nissan Chemical's Alumina Sol #200 with $Al_2O_3$ = 10 wt.%) serving as a binder, and granulated into 0.15 to 0.5 mmφ granules. The NaY-type zeolite granules are dried at 120°C, and then baked at 500°C for 2 hours. With an aqueous solution of silver nitrate that corresponds to 50% (in terms of the metal by mol) of the Na cation site of Na-Y, the zeolite granules are ion-exchanged (solid/liquid ratio, 3.0 liters/kg) by keeping them therein at room temperature for 30 minutes and then heating them at 85°C for 1 hour. These are then fully washed with distilled water, dried at 120°C, and then baked at 500°C for 2 hours.

Using the adsorbent (Ag-Na-Y) together with 2,4-xylidine serving as a desorbent, a mixture of α-picoline:β-picoline:2,6-lutidine:2,4-lutidine = 1:1:1:1 (by weight) is processed for adsorptive separation in the same manner as in Example 2.

The desired product, 2,6-lutidine, is taken out as a raffinate flow along with the desorbent; and the remaining 2,6-lutidine not having been recovered in the raffinate flow and the other components α-picoline, β-picoline and 2,4-lutidine are taken out as an extract flow along with the desorbent.

An index of the separation capability of the system is the adsorption recovery for 99.5 % product purity to be defined by the following equation, and its time-dependent change is plotted, based on the adsorption recovery at the start of the system.

Adsorption Recovery

= [(amount of 2,6-lutidine recovered as product, g/hr)/(amount of 2,6-lutidine in substituted pyridine mixture processed for adsorptive separation, g/hr)] x 100

In this Example, a part of the desorbant is continuously extracted out and processed in the distillation units 3 and 4 to remove the impurities from it, and then circulated and reused in the system. Accordingly, as shown in Fig. 7, the adsorption capability of the system is not lowered.

Comparative Example 5:

The same absorptive separation process as in Example 3 is repeated herein. In this process, however, the step of continuously extracting apart of the desorbent to be circulated and reused in the system, and processing it for removing impurities therefrom through distillation is omitted. The adsorption recovery in the system is greatly lowered, as shown in Fig. 7.

## Claims

1. A method for producing an isomer of an aromatic compound substituted with alkyl group(s) and/or halogen atom (s), by adsorptive separation using a zeolite-containing adsorbent and a desorbent, **characterised in that** the desorbent is, after having been processed to remove impurities from it, said impurities having a higher boiling point than the desorbent, supplied to the adsorptive separation step.

2. The method for producing an isomer of an aromatic compound as claimed in claim 1, wherein the aromatic compound has a benzene ring or heterocyclic ring structure.

3. The method for producing an isomer of an aromatic compound as claimed in claim 1 or 2, wherein the aromatic compound is ring-substituted with at least one halogen element.

4. The method for producing an isomer of an aromatic compound as claimed in any of claims 1 to 3, wherein the impurities in the desorbent are removed through any of distillation, purging or adsorption to a solid adsorbent.

5. The method for producing an isomer of an aromatic compound as claimed in any of claims 1 to 3, wherein the impurities in the desorbent are removed by replacing a part of the used desorbent with an impurity-free fresh desorbent.

6. The method for producing an isomer of an aromatic compound as claimed in any of claims 1 to 5, wherein all or part of the desorbent to be supplied to the adsorptive separation step is first continuously or intermittently supplied to a step of removing impurities from it, and then supplied to the adsorptive separation step.

7. A method according to any one of claims 1 to 6, which method comprises the steps of introducing a starting material into an adsorption chamber through which a desorbent is flowing, withdrawing from the adsorption chamber a raffinate of unabsorbed starting material and a proportion of desorbent, withdrawing remaining desorbent and desired isomer from an end of the adsorption chamber and recirculating it to the opposite end, after passage through a section of the adsorption chamber extracting from the adsorption chamber an extract of the desired isomer and a

proportion of desorbent, subjecting each of the extract and raffinate to respective separation processes to recover the desired isomer from the extract, unabsorbed starting material from the raffinate and respective proportions of the desorbent from each of the extracts and raffinate, subjecting desorbent from the extract and raffinate to a separation process to remove impurities therefrom and recirculating the purified desorbent to the adsorption chamber.

**8.** A method according to claim 7, wherein the starting material is a mixture of ethyl benzene and o-, m- and p-xylenes, the desired isomer is p-xylene and the desorbent comprises at least 90 wt.% of p-diethylbenzene.

**9.** A method according to any one of claims 1 to 6, which method comprises the steps of introducing a starting material into an adsorption chamber through which a desorbent is flowing, withdrawing from the adsorption chamber a raffinate of the desired isomer and a proportion of desorbent, withdrawing remaining desorbent and starting material from an end of the adsorption chamber and recirculating it to the opposite end, after passage through a section of the adsorption chamber extracting from the adsorption chamber an extract of remaining starting material and a proportion of desorbent, subjecting each of the extract and raffinate to respective separation processes to recover the desired isomer from the raffinate, unabsorbed starting materials from the extract and respective proportions of the desorbent from each of the extract and raffinate, subjecting desorbent from the extract and raffinate to a separation process to remove impurities therefrom and recirculating the purified desorbent to the adsorption chamber.

**10.** A method according to claim 9, wherein the starting material is a mixture of o-, m- and p-dichlorobenzene, the desired isomer is m-chlorobenzene and the desorbent is 3,4-dichlorotoluene.

**11.** A method according to any one of the previous claims, wherein the desorbent is distilled to remove from it impurities having a lower boiling point than the desorbent and impurities having a higher boiling point than the desorbent by evaporation.

**12.** A method according to any one of the previous claims, wherein the desorbent is processed to remove one of more of the following impurities: water, hydrochloric acid, phenols, dimers of aromatic compounds, unsaturated hydrocarbon-containing compounds and oxygen-containing compounds having aldehyde groups and carboxyl groups.

## Patentansprüche

**1.** Verfahren zur Herstellung eines Isomers einer aromatischen Verbindung, die mit Alkylgruppe(n) und/oder Halogenatom(en) substituiert ist, durch adsorptive Trennung unter Verwendung eines zeolithhältigen Adsorptionsmittels und eines Desorptionsmittels, **dadurch gekennzeichnet, dass** das Desorptionsmittel, nachdem es prozessiert wurde, um Verunreinigungen daraus zu entfernen, wobei die Verunreinigungen einen höheren Siedepunkt aufweisen als das Desorptionsmittel, dem Schritt der adsorptiven Trennung zugeführt wird.

**2.** Verfahren zur Herstellung eines Isomers einer aromatischen Verbindung nach Anspruch 1, worin die aromatische Verbindung eine Benzolring- oder eine heterozyklische Ringstruktur aufweist.

**3.** Verfahren zur Herstellung eines Isomers einer aromatischen Verbindung nach Anspruch 1 oder 2, worin die aromatische Verbindung mit zumindest einem Halogenelement ringsubstituiert ist.

**4.** Verfahren zur Herstellung eines Isomers einer aromatischen Verbindung nach einem der Ansprüche 1 bis 3, worin die Verunreinigungen im Desorptionsmittel durch eines von Destillation, Spülen oder Adsorption an ein festes Adsorptionsmittel entfernt werden.

**5.** Verfahren zur Herstellung eines Isomers einer aromatischen Verbindung nach einem der Ansprüche 1 bis 3, worin die Verunreinigungen im Desorptionsmittel entfernt werden, indem ein Teil des verwendeten Desorptionsmittels durch ein verunreinigungsfreies frisches Desorptionsmittel ersetzt wird.

**6.** Verfahren zur Herstellung eines Isomers einer aromatischen Verbindung nach einem der Ansprüche 1 bis 5, worin das gesamte, dem Schritt der adsorptiven Trennung zuzuführende Desorptionsmittel oder ein Teil davon zunächst kontinuierlich oder periodisch einem Schritt der Entfernung von Verunreinigungen daraus und dann erst dem Schritt der adsorptiven Trennung zugeführt wird.

**7.** Verfahren nach einem der Ansprüche 1 bis 6, wobei das Verfahren die folgenden Schritte umfasst: das Einführen

eines Ausgangsmaterials in eine Adsorptionskammer, durch die ein Desorptionsmittel fließt, das Abziehen eines Raffinats von nicht absorbiertem Ausgangsmaterial und eines Anteils des Desorptionsmittels aus der Adsorptionskammer, das Abziehen des verbleibenden Desorptionsmittels und eines gewünschten Isomers von einem Ende der Adsorptionskammer und das Rückführen desselben zum entgegengesetzten Ende, das Extrahieren eines Extrakts des gewünschten Isomers und eines Anteils des Desorptionsmittels aus der Adsorptionskammer nach Verlauf durch einen Abschnitt der Adsorptionskammer, das jeweilige Aussetzen des Extrakts bzw. des Raffinats gegenüber Trennverfahren, um das gewünschte Isomer aus dem Extrakt, nicht absorbiertes Ausgangsmaterial aus dem Raffinat und die jeweiligen Anteile des Desorptionsmittel aus Extrakten und Raffinat zu gewinnen, das Aussetzen des Desorptionsmittels aus dem Extrakt und dem Raffinat gegenüber einem Trennverfahren, um Verunreinigungen daraus zu entfernen, und das Rückführen des gereinigten Desorptionsmittels in die Adsorptionskammer.

**8.** Verfahren nach Anspruch 7, worin das Ausgangsmaterial ein Gemisch aus Ethylbenzol und o-, m- und p-Xylolen ist, das gewünschte Isomer p-Xylol ist und das Desorptionsmittel zumindest 90 Gew.-% p-Diethylbenzol umfasst.

**9.** Verfahren nach einem der Ansprüche 1 bis 6, wobei das Verfahren die folgenden Schritte umfasst: das Einführen eines Ausgangsmaterials in eine Adsorptionskammer, durch die ein Desorptionsmittel fließt, das Abziehen eines Raffinats des gewünschten Isomers und eines Anteils des Desorptionsmittels aus der Adsorptionskammer, das Abziehen des verbleibenden Desorptionsmittels und Ausgangsmaterials von einem Ende der Adsorptionskammer und das Rückführen desselben zum entgegengesetzten Ende, das Extrahieren eines Extrakts des verbleibenden Ausgangsmaterials und eines Anteils des Desorptionsmittels aus der Adsorptionskammer nach Verlauf durch einen Abschnitt der Adsorptionskammer, das jeweilige Aussetzen des Extrakts bzw. des Raffinats gegenüber Trennverfahren, um das gewünschte Isomer aus dem Raffinat, nicht absorbiertes Ausgangsmaterial aus dem Extrakt und die jeweiligen Anteile des Desorptionsmittel aus dem Extrakt und Raffinat zu gewinnen, das Aussetzen des Desorptionsmittels aus dem Extrakt und dem Raffinat gegenüber einem Trennverfahren, um Verunreinigungen daraus zu entfernen, und das Rückführen des gereinigten Desorptionsmittels in die Adsorptionskammer.

**10.** Verfahren nach Anspruch 9, worin das Ausgangsmaterial ein Gemisch aus o-, m- und p-Dichlorbenzol ist, das gewünschte Isomer m-Chlorbenzol ist und das Desorptionsmittel 3,4-Dichlortoluol ist.

**11.** Verfahren nach einem der vorangegangenen Ansprüche, worin das Desorptionsmittel destilliert wird, um daraus Verunreinigungen mit einem geringeren Siedepunkt als das Desorptionsmittel und Verunreinigungen mit einem höheren Siedepunkt als das Desorptionsmittel durch Verdampfen zu entfernen.

**12.** Verfahren nach einem der vorangegangenen Ansprüche, worin das Desorptionsmittel prozessiert wird, um daraus eine oder mehrere der folgenden Verunreinigungen zu entfernen: Wasser, Salzsäure, Phenole, Dimere aromatischer Verbindungen, ungesättigte Kohlenwasserstoffe enthaltende Verbindungen und sauerstoffhältige Verbindungen mit Aldehydgruppen und Carboxylgruppen.

## Revendications

**1.** Procédé de production d'un isomère d'un composé aromatique substitué par un ou plusieurs groupes alkyle et/ou un ou plusieurs atomes d'halogène, par séparation adsorptive en utilisant un adsorbant contenant une zéolite et un désorbant, **caractérisé en ce que** le désorbant est, après avoir été traité pour en éliminer les impuretés, lesdites impuretés ayant un point d'ébullition plus élevé que le désorbant, approvisionné vers l'étape de séparation adsorptive.

**2.** Procédé de production d'un isomère d'un composé aromatique selon la revendication 1, dans lequel le composé aromatique comprend une structure cyclique benzénique ou hétérocyclique.

**3.** Procédé de production d'un isomère d'un composé aromatique selon la revendication 1 ou 2, dans lequel le composé aromatique est substitué sur le cycle par au moins un élément halogéné.

**4.** Procédé de production d'un isomère d'un composé aromatique selon l'une quelconque des revendications 1 à 3, dans lequel les impuretés dans le désorbant sont éliminées par distillation, purge ou adsorption sur un adsorbant solide.

**5.** Procédé de production d'un isomère d'un composé aromatique selon l'une quelconque des revendications 1 à 3, dans lequel les impuretés dans le désorbant sont éliminées en remplaçant une partie du désorbant utilisé par un

désorbant frais sans impuretés.

6. Procédé de production d'un isomère d'un composé aromatique selon l'une quelconque des revendications 1 à 5, dans lequel tout ou une partie du désorbant à approvisionner dans l'étape de séparation adsorptive est d'abord approvisionné(e) de manière continue ou intermittente vers une étape d'élimination des impuretés, puis approvisionné(e) vers l'étape de séparation adsorptive.

7. Procédé selon l'une quelconque des revendications 1 à 6, lequel procédé comprend les étapes consistant à introduire une matière première dans une chambre d'adsorption à travers laquelle s'écoule un désorbant, retirer de la chambre d'adsorption un raffinat de matière première non adsorbée et d'une proportion de désorbant, retirer le désorbant restant et l'isomère souhaité par une extrémité de la chambre d'adsorption et les faire recirculer par l'autre extrémité, après le passage à travers une section de la chambre d'adsorption, extraire de la chambre d'adsorption un extrait de l'isomère souhaité et d'une proportion de désorbant, soumettre chacun de l'extrait et du raffinat à des processus de séparation respectifs pour récupérer l'isomère souhaité de l'extrait, la matière première non absorbée du raffinat et des proportions respectives du désorbant de chacun de l'extrait et du raffinat, soumettre le désorbant provenant de l'extrait et du raffinat à un processus de séparation pour en éliminer les impuretés et faire recirculer le désorbant purifié vers la chambre d'adsorption.

8. Procédé selon la revendication 7, dans lequel la matière première est un mélange d'éthylbenzène et de o-, met p-xylènes, l'isomère désiré est le p-xylène et le désorbant comprend au moins 90 % en poids de p-diéthylbenzène.

9. Procédé selon l'une quelconque des revendications 1 à 6, lequel procédé comprend les étapes consistant à introduire une matière première dans une chambre d'adsorption à travers laquelle s'écoule un désorbant, retirer de la chambre d'adsorption un raffinat de l'isomère désiré et d'une proportion de désorbant, retirer le désorbant et la matière première restants par une extrémité de la chambre d'adsorption et les faire recirculer par l'autre extrémité, après le passage à travers une section de la chambre d'adsorption extraire de la chambre d'adsorption un extrait de matière première restante et une proportion de désorbant, soumettre chacun de l'extrait et du raffinat à des processus de séparation respectifs pour récupérer l'isomère souhaité du raffinat, la matière première non absorbée de l'extrait et des proportions respectives du désorbant de chacun de l'extrait et du raffinat, soumettre le désorbant provenant de l'extrait et du raffinat à un processus de séparation pour en éliminer les impuretés et faire recirculer le désorbant purifié vers la chambre d'adsorption.

10. Procédé selon la revendication 9, dans lequel la matière première est un mélange de o-, m- et p-dichlorobenzènes, l'isomère désiré est le m-dichlorobenzène et le désorbant est le 3,4-dichlorotoluène.

11. Procédé selon l'une quelconque des revendications précédentes, dans lequel le désorbant est distillé pour en éliminer les impuretés ayant un point d'ébullition inférieur à celui du désorbant et les impuretés ayant un point d'ébullition supérieur à celui du désorbant par évaporation.

12. Procédé selon l'une quelconque des revendications précédentes, dans lequel le désorbant est traité pour éliminer une ou plusieurs des impuretés suivantes : eau, acide chlorhydrique, phénols, dimères de composés aromatiques, composés contenant des hydrocarbures insaturés et composés contenant de l'oxygène comprenant des groupes aldéhyde et carboxyle.

Fig. 1

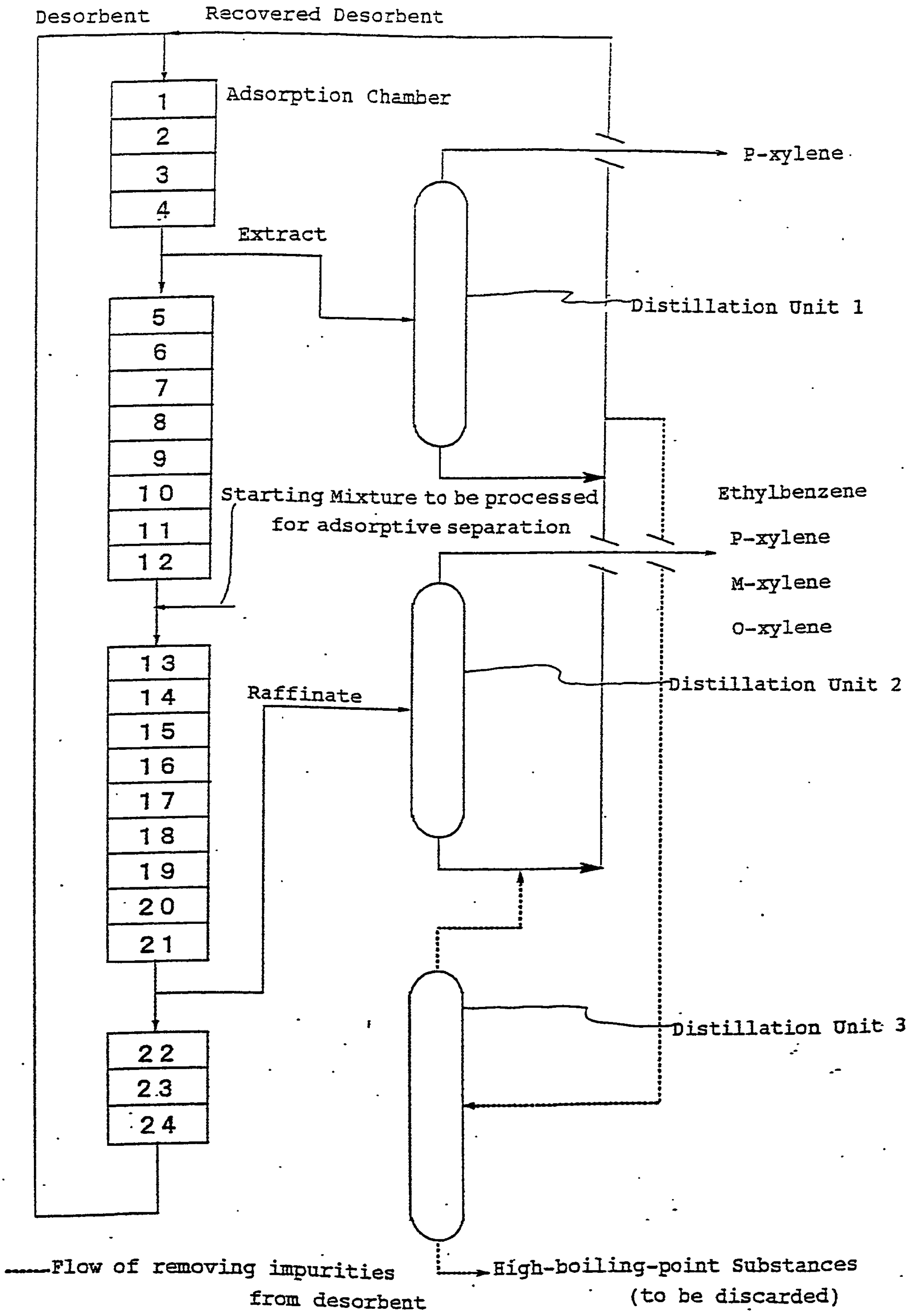
Desorbent
Recovered Desorbent
Adsorption Chamber
1
2
3
4
5
6
7
8
9
10
11
12
13
14
15
16
17
18
19
20
21
22
23
24
Extract
P-xylene
Distillation Unit 1
Starting Mixture to be processed
for adsorptive separation
Ethylbenzene
P-xylene
M-xylene
O-xylene
Raffinate
Distillation Unit 2
Distillation Unit 3
Flow of removing impurities
from desorbent
High-boiling-point Substances
(to be discarded)

Fig. 2

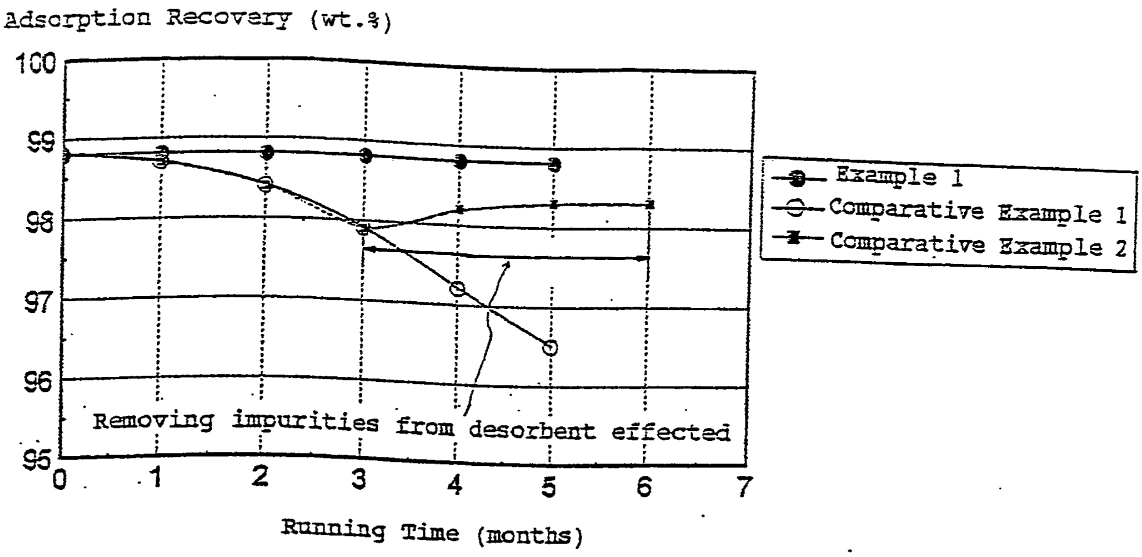
Adsorption Recovery (wt.%)
100
99
98
97
96
95
0
1
2
3
4
5
6
7
Example 1
Comparative Example 1
Comparative Example 2
Removing impurities from desorbent effected
Running Time (months)

Fig. 3

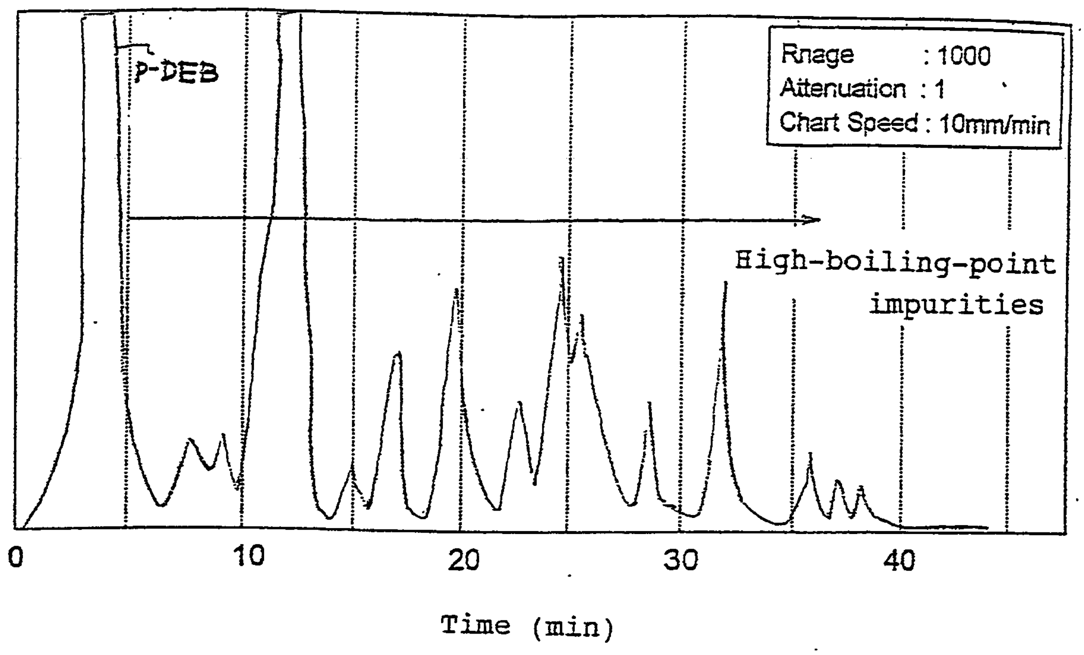
P-DEB
Rnage : 1000
Attenuation : 1
Chart Speed : 10mm/min
High-boiling-point impurities
0
10
20
30
40
Time (min)

Fig. 4

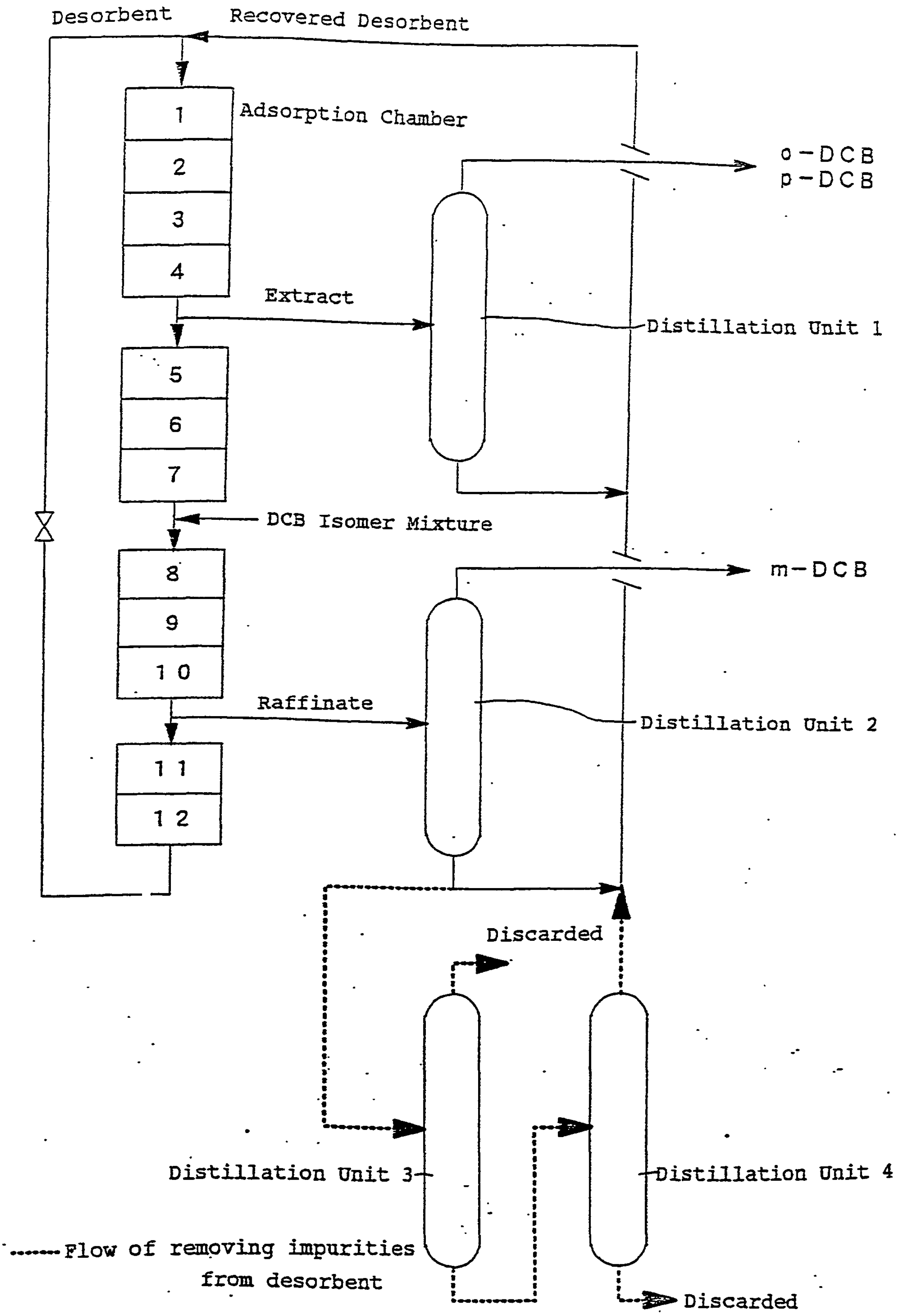
Desorbent
Recovered Desorbent
1
2
3
4
Adsorption Chamber
o-DCB
p-DCB
Extract
Distillation Unit 1
5
6
7
DCB Isomer Mixture
8
9
10
m-DCB
Raffinate
Distillation Unit 2
11
12
Discarded
Distillation Unit 3
Distillation Unit 4
Flow of removing impurities from desorbent
Discarded

Fig. 5

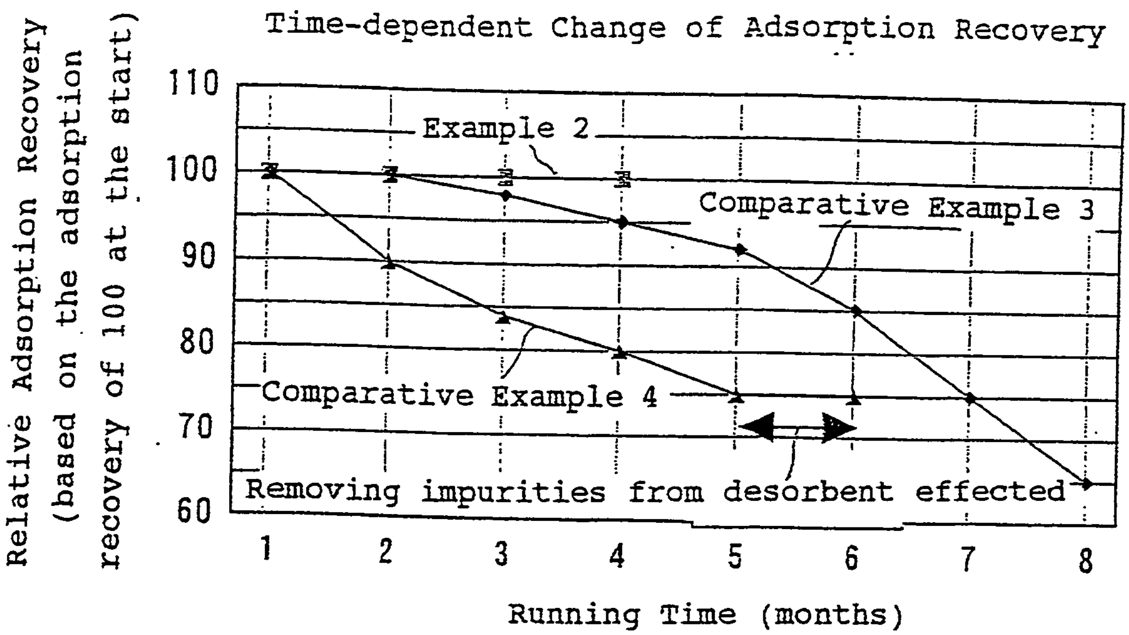
Time-dependent Change of Adsorption Recovery
Relative Adsorption Recovery (based on the adsorption recovery of 100 at the start)
110
100
90
80
70
60
Example 2
Comparative Example 3
Comparative Example 4
Removing impurities from desorbent effected
1
2
3
4
5
6
7
8
Running Time (months)

Fig. 6

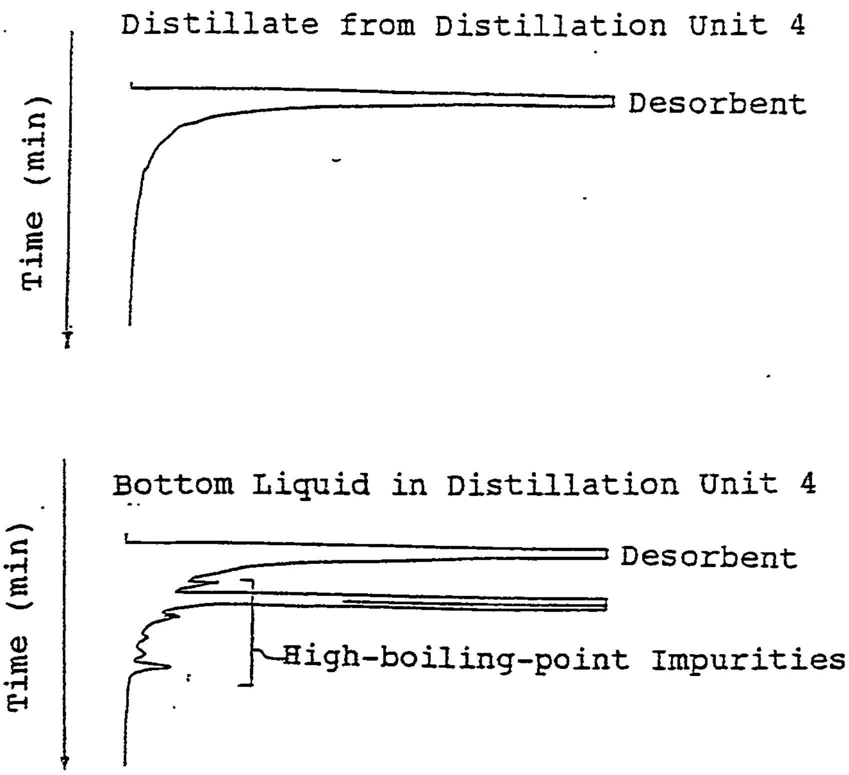
Distillate from Distillation Unit 4
Desorbent
Time (min)
Bottom Liquid in Distillation Unit 4
Desorbent
High-boiling-point Impurities
Time (min)

Fig. 7 Time-dependent Change of Adsorption Recovery

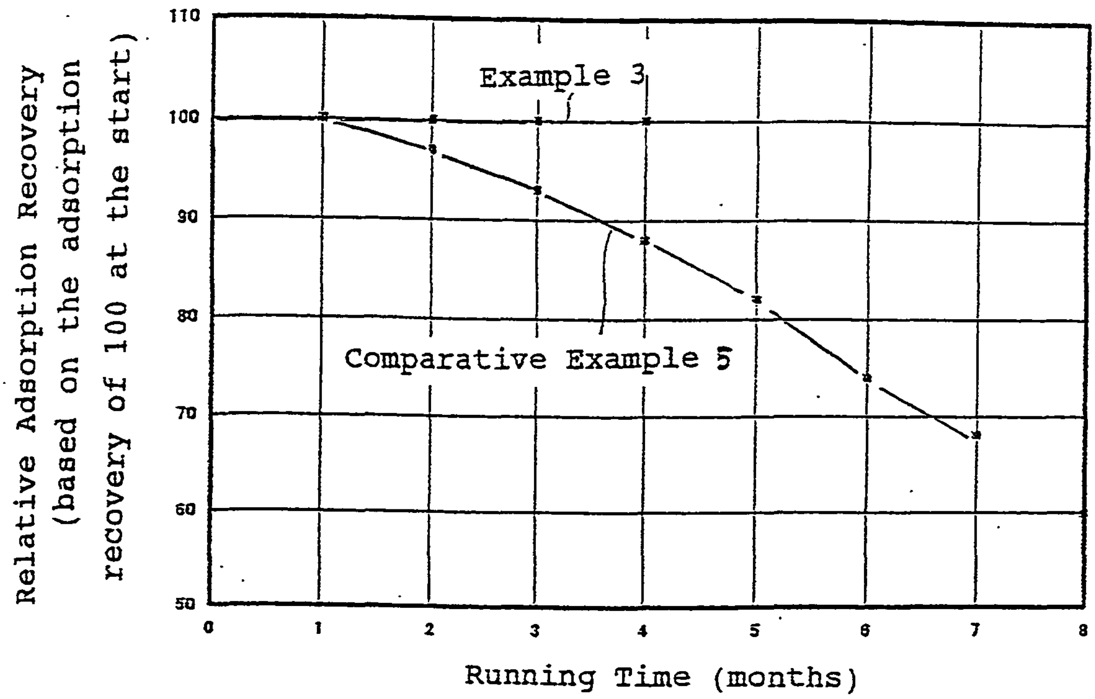

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 1045457 A
- JP 4330025 A
- JP 4046933 A
- JP 9143162 A
- JP 10251230 A
- EP 173440 A
- EP 425740 A
- EP 0412215 A
- JP 48015833 A